# EUROPEAN PATENT APPLICATION

(11) **EP 2 425 770 A1**
(43) Date of publication of application: **07.03.2012**
(21) Application number: 10009131.3
(22) Date of filing: 02.09.2010
(51) Int. Cl.: A61B 5/07

(54) **Implantable device for real-time monitoring of glycemia and dosage**

(71) Applicant: Castro Dourado Pinezi, Juliana, Goiania Goias AC (BR)
(72) Inventor: Castro Dourado Pinezi, Juliana, Goiania Goias AC (BR)
(74) Representative: Lorente Berges, Ana

(57) **Abstract**

The present invention refers to an implantable device for constant collection and dosage of glycemia, with real-time monitoring, with no need of external collection by punctioning which is constituted by a metal Compartment (01), hermetically sealed with the Cap (02), containing at least a Micro computerized mini-center (08) fed by the Battery (12) or similar, which is externally rechargeable; it will also contain a Sensor (11) which is responsible to collect a portion of the circulating blood and read glycemia levels in real time, sending reading information through the transmission component (10), said Implantable Device will be connected to an inlet Catheter (04) with its collecting Edge (03) duly implanted by means of punctioning at the Subclavian Vein (VS), so to deviate a small flow of blood to pass through within said Dosage and Monitoring Device, thus allowing the Sensor (11) to verify the glycemic dosage and following its natural course by exiting the exit Catheter (06) through the outlet Edge (05), thus returning to the blood current within said Subclavian Vein (VS).

## Description

The present invention refers to an implantable device for constant collection and dosage of glycemia, with real-time monitoring, with no need of external collections by vein puncture.

Scientific studies prove that the level of glucose is determined by using vein plasma or serum, as measured by the glucose oxidase method as used in laboratories. For clinical purposes, both the level in plasma and in serum are similar enough to be interchangeable. However, currently availble glucose meters measuring capillary glycemia only guarantee 15% exact results over the values as measured in plasma or serum by clinical laboratories.

The collection of peripheral blood for laboratory tests of glycemia dosage is made at the surface veins of upper members, usually at the basilic vein or the cephalic vein. The dosage on that blood is considered as the golden standard to which glucose meter readings are compared for a periodical quality control, usually each six months. Brachial veins are joined to the basilic vein to form the auxiliary vein, which the other surface vein of the upper member (cephalic vein) flows to. The auxiliary vein becomes the subclavian vein after crossing the first rib. For having a larger diameter, for being more easily accessed in surgeries and for being the first choice when a central vein access is indicated (since this is the site with less risk of thrombosis and infections related to catheters in comparison with internal femoral and jugular veins), the subclavian vein is the ideal candidate for the implantation of implantable catheters of the device at issue. Therefore, the reading of the device as presented herein will be made by using the same blood collected in a laboratory and considered as a golden standard for the dosage of glycemia.

The use of new technologies in the monitoring of glycemia has been a challenge for specialists and corporations. Many of them do not hold a license in some countries, while others have been taken from the market when they started to be used, due to problems that were verified. An example was the quick commercialization of the Pendra device, which was subsequently taken off from the European market Wentholt et al disclosed in an article (Diabctologia 2005; 48: 1055-1058) what Would be the development of a non-invasive glycemia sensor, which turned out to be a danger for diabetic patients since its average reading error was 52%.

Another non-invasive device having problems with humidity which was taken off from the market was Cygnus Glucowatch.

Currently, one equipment meeting clinical interests is Medtronic Diabetes MiniMed CGMS - continuous glucose monitoring. That device uses a needle to measure glucose in the interface by means of the reaction between glucose-oxidase and the immobilized enzyme in the electrode as subcutaneously inserted, enabling the observation of fluctuating rates of glycemia throughout the day. The sensor lasts about three days and receives the results, downloading them to make registration comparisons. In some cases, they are used for seven days. The values are joined and sum a total each five minutes. The device has a small electrode connected to a router downloading data to another equipment. When installed, it needs one hour of pre-heating to then obtain a glycemia value to be included. There are about 280 measurements per day. The patient makes a log with feeding and carbohydrate counts to enable the doctor to evaluate the reason of oscillations in glycemia. The use of CGMS serves to improve glycemia control, detect and reduce the risk of hypoglycemic events and improve intensive insulin delivery schemes. However, considering the discomfort produced and how it is implanted, it cannot be used for long periods to substitute traditional auto monitoring with the glucose meter examining the blood drop as obtained by the lancet at the edge of the fingers (capillary glycemia). In the same way, Medtronic MiniMed long-term IV sensor is still under development, having an intravessel electrode linked to an insulin pump.

Equally, another available subcutaneous glucose sensor is GlucoDay sensor (Menarini Diagnostics, Firenze, Italy). Said sensor is based on microdyalisis, wherein the catheter is connected to a walkman-sized device containing a glucose biosensor. Glucose is reported each three minutes.

On the other hand, HypoMon is just a system for hypoglycemia detection and alarm (glycemia < 45 mg/dl). The device uses four electrodes in contact with the skin and a handheld computer with wireless communication system (no wires). It continuously monitors heart frequency and QT intervals and detects physiological signs of hypoglycemia by means of an algorithm.

In the same fashion, a device known as Freestyle Navigator is also known, using a subcutaneous needle and a transmitting device fixed to the skin by a sticker held for various days. By means of wireless communication, it sends the dosage of glycemia minute by minute to a pager-shaped receiver. There is also the DexCom implantable system, wherein the sensor is installed subcutaneously and the data is sent to an external receiver. However, these options are still in clinical evaluation or development and are inconveniently painful and/or expose the diabetic patient to the society as an ill individual with various accessories fixed to the body, limiting its privacy.

Furthermore, even among so many options, no device has yet proved to be sufficiently accurate to substitute auto monitoring with glucose meters. Diabetic patients remain depending on said auto monitoring measuring capillary glycemia by using lancets damaging finger edges to obtain blood drops used for analysis. These patients consider the method as painful, constraining, expensive, anti-hygienic and inconvenient, as well as the traditional methods causing large discomfort to patients.

With the purpose to solve such inconveniences and difficulties, the present invention has been developed and will be better understood according to the attached figures, wherein:
Figure 1 shows a front view of the **"IMPLANTABLE DEVICE FOR REAL-TIME MONITORING AND DOSAGE OF GLYCEM**IA", duly implanted to the subclavian vein.
Figure 2 shows the same Figure 1, but with the **"IMPLANTABLE DEVICE FOR REAL-TIME MONITORING AND DOSAGE OF GLYCEMIA"** in an internal view of said Device.

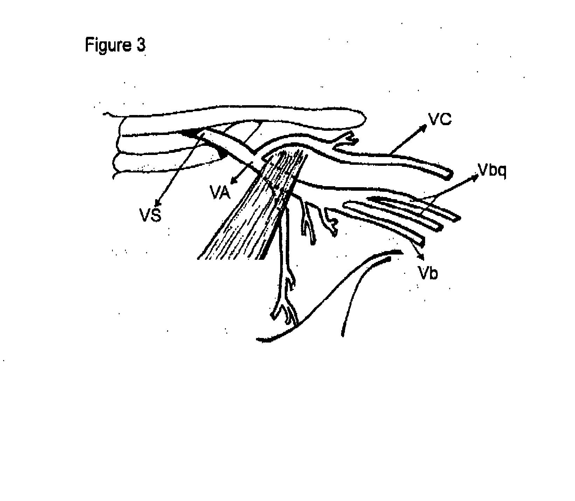

Figure 3 shows a set of views, among which we can mention the Subclavian Vein (VS) itself, wherein the Device of the present claim should be implanted, as well as the Axillary (VA) and Cephalic (VC) veins. besides the Basilic Vein (Vb) and Brachial Veins (Vbq).

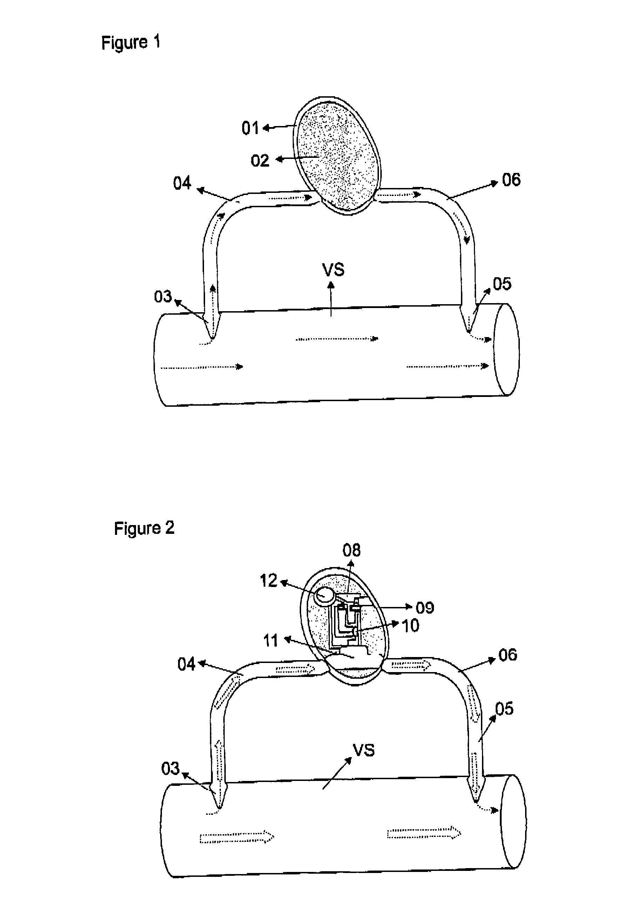

As we can see on Figures 1 and 2, the "IMPLANTABLE DEVICE FOR REAL-TIME MONITORING AND DOSAGE OF GLYCEMIA" comprises a metal compartment (01), hermetically sealed with the Cap (02), containing at least one micro computerized Minicentral (08) fed by the Battery (12) or similar, externally rechargeable, and will also contain a Sensor (11) which is responsible to effect glycemia readings in real time, by sending reading information by means of the transmission component (10).

With that purpose, said Implantable Device Will be connected to an inlet catheter (04) with its collecting Edge (03), which will be implanted by means of punctioning at the Subclavian Vein (VS), so to deviate a small flow of blood which will pass through within said Dosage and Monitoring Device, thus allowing the Sensor (11) to verify the glycemia dosage and follow its natural course, exiting through the outlet catheter (06) at the outlet edge (05), thus returning to the blood flow inside said Subclavian Vein (VS).

Therefore, the external components which will remain in contact with the internal organs of the patient will be manufactured and covered with titanium or another similar metal to vessel prosthesis allowing for long use for various years and presenting less risk of rejection.

We should strongly highlight that the result of glycemia analysis as effected by said Device will be sent by means of radio frequency waves (or similar) to an external receiver, which may have various shapes and sizes, preferably that of an object for personal use, such as a wristwatch, wherein the patient will be able to receive and monitor glycemia data as continuously measured, minute by minute, besides containing the schedule to advise the patient, by means of an alarm signal, in case of any abnormal situation, be it in case of hypoglycemia or even hyperglycemia, since the micro computerized center (08) will be scheduled with minimum and maximum dosage scales for glycemia, so to send a warning to the receiver and, therefore, the patient may take his or her decisions.

## Claims

1. **"IMPLANTABLE DEVICE FOR REAL-TIME MONITORING AND DOSAGE OF GLYCEMIA"** for constant dosage of glycemia, with real-time monitoring, **characterized by** comprising a hermetically sealed Compartment (01) with the Cap (02), containing at least a Micro computerized minicenter (08) supplied by the Battery (12) or similar which is externally rechargeable, and will also contain a Sensor (11) for reading and analysis and a transmitting Component (10) to send the data through wireless waves.

2. according to claim 1, **characterized by** reading glycemia in real time, with no discharge of blood, directly collected by an inlet Catheter (04) with a collecting Edge (03), duly implanted by means of puncture in the Subclavian Vein (VS) so to deviate a small flow of blood to pass through within said Monitoring and Dosage Device, thus allowing the Sensor (11) to verify glycemia dosage and allowing the blood to follow its natural course exiting through the outlet Catheter (06) through the outlet Edge (05), thus returning to the blood flow inside said Subclavian Vein (VS).

3. according to claim 1 and 2, **characterized by** containing a data reception device allowing the patient to both monitor, minute by minute, the glycemia rate and be advised by means of a sound or visual alarm in case of any change indicating hypoglycemia or even hyperglycemia.
